# EUROPEAN PATENT SPECIFICATION

(11) **EP 1 450 691 B1**
(45) Date of publication and mention of the grant of the patent: **23.03.2011**
(21) Application number: 02786469.3
(22) Date of filing: 22.10.2002
(51) Int. Cl.: A61B 17/04, A61B 17/06, A61B 17/10

(54) **WOUND SUTURING DEVICE**
WUNDNÄHVORRICHTUNG
DISPOSITIF DE SUTURE DE PLAIES

(30) Priority: 22.10.2001 US 345212 P
(43) Date of publication of application: 01.09.2004
(73) Proprietor: Interventional Therapies, LLC, Westport, Connecticut 06880 (US)
(72) Inventor: SHIKHMAN, Oleg, Trumbull, CT 06611 (US); SCIRICA, Paul, A., Huntington, CT 06484 (US)
(74) Representative: Kayser, Martin
(86) International application number: PCT/US2002/033747
(87) International publication number: WO 2003/034924

(56) References cited:
- WO-A-01/35833
- US-A- 5 431 666
- US-A- 5 725 536
- US-A- 5 766 183
- US-A- 5 792 153
- US-A- 5 797 927

## Description

### BACKGROUND

When performing catheterization procedures, such an angiography or angioplasty, a catheter is generally introduced percutaneously (i.e., through the skin) into the vascular system by first penetrating the skin and underlying tissue, and then the blood vessel with a sharpened hollow needle. Location of a blood vessel, such as an artery, is typically achieved by feeling for the pulse, since such structures usually cannot be seen through the skin. Next, a guide wire is commonly inserted through the lumen of the hollow needle and is caused to enter the selected blood vessel. Subsequently, the needle is typically slid off the guide wire and a combination of a dilator and sheath are fed over the guide wire and pushed through the skin to enter the vessel. The guide wire and dilator can then be removed, and the desired catheter used to carry out the procedure is fed through the lumen of the sheath and advanced through the vascular system until the working end of the catheter is appropriately positioned. Following the conclusion of the catheterization procedure, the working catheter will be withdrawn and, subsequently, the sheath can also be removed from the wound, or left in place to facilitate closure.

At this point in the procedure, the vessel leakage is controlled in order to stem the flow of blood through the puncture. Because it is common practice to administer a blood thinning agent to the patient prior to many of the catheterization procedures, stemming the blood flow can be troublesome. A common method of sealing the wound is to maintain external pressure over the vessel until the puncture naturally seals. This method of puncture closure typically takes at least thirty minutes, with the length of time usually being substantially greater if the patient is hypertensive or anti-coagulated. In some anti-coagulated patients, the sheath is left in place for hours to allow the anticoagulant to wear off. When human hand pressure is utilized, it can be uncomfortable for the patient and can use costly professional time on the part of the hospital staff. Other pressure techniques, such as pressure bandages, sandbags or clamps, have been employed, but these devices also require the patient to remain motionless for an extended period of time and the patient must be closely monitored to ensure their effectiveness.

Document WO 01/35833 A1 discloses a suturing apparatus according to the preamble of the appended claim 1.

There remains a need in the art for effective percutaneous tissue closure that is quick, easy to instruct and easy to learn, effective and comfortable for the patient.

### SUMMARY

The above described and other disadvantages of the prior art are overcome and alleviated by the present wound suturing device, comprising a housing and an elongated shaft connected thereto and two needles within the shaft, the two needles configured to travel distally across two tissue engaging gaps within a tissue engaging section positioned distally from the housing on said shaft, wherein each tissue receiving gap has two opposing surfaces into which one side of a wound can be received, wherein each gap is shaped to have a depth to facilitate the placement of the edge of a wound therein such that at least one surface comprises a stop surface, wherein the stop surface is squared in relation to the longitudinal axis of the tissue engaging section at a middle portion thereof to provide good tactile feel to the surgeon when tissue is engaged.

The above described and other disadvantages of the prior art are also overcome and alleviated by the present trigger mechanism for actuating both needles, the trigger mechanism configured to prevent incomplete needle actuation, the trigger mechanism comprising a housing and a trigger configured to move relative to said housing, two track means positioned on one of the trigger and the housing, the two track means including a first end portion and a second end portion and a plurality of ratchet teeth between the first and second end portions, and a pin means slideable within the track means as the trigger is actuated in a first direction, the pin means engaging the ratchet teeth as the trigger is actuated, wherein each of the ratchet teeth prevent movement of said trigger in a second direction as the ratchet teeth engage the pin means.

The above described and other disadvantages of the prior art are also overcome and alleviated by the present automatic needle sector mechanism, configured relative to and including a needle trigger mechanism, the automatic needle selector mechanism comprising a trigger mechanism movable relative to a housing, the trigger mechanism engageable with a selector arm such that the engaged selector arm is in a first position prior to actuation of the trigger mechanism, and wherein the engaged selector arm is in a second position subsequent to actuation of the trigger mechanism.

The above described and other features are exemplified by the following figures and detailed description.

### BRIEF DESCRIPTION OF THE DRAWINGS

Referring now to the figures wherein the like elements are numbered alike:
FIGURE 1 is a side plan view of the an exemplary wound suturing apparatus;
FIGURE 2 is a bottom perspective view of an exemplary retainer member;
FIGURES 3-5 illustrate perspective views of an exemplary actuation mechanism;
FIGURE 6 is a perspective view of an exemplary slip-free mechanism;
FIGURES 7 and 8 are cross sectional and perspective views, respectively, of an exemplary tissue engagement section;
FIGURE 9 is a cross sectional view of an exemplary suture and ferrules; and
FIGURE 10 is a cross sectional view of an exemplary needle and spherical member.

### DETAILED DESCRIPTION

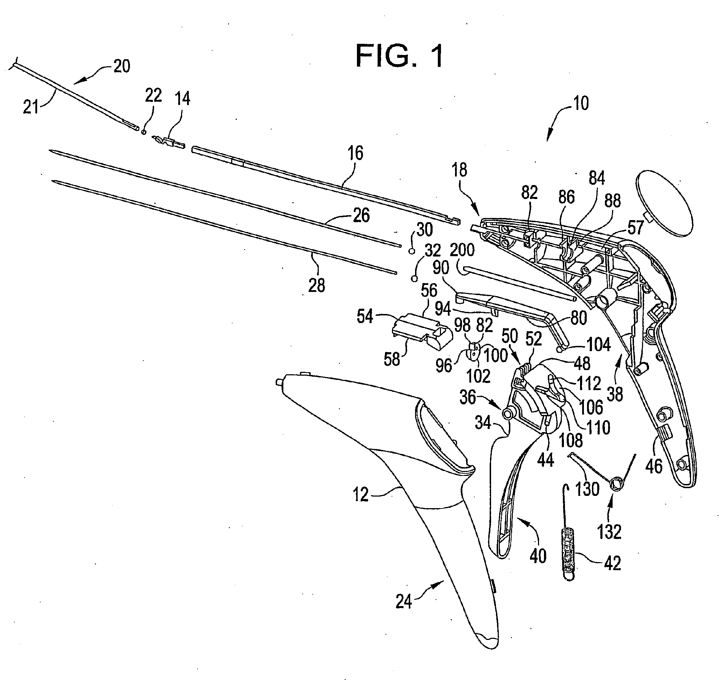

Referring to the drawings, wherein like reference numerals identify similar or identical elements throughout the several views, an apparatus for applying a suture to body tissue is illustrated in FIGURE 1 and is designated generally by reference numeral 10. Note that the terms "first" and "second" as used herein are for the reader's convenience and should not be interpreted as necessarily denoting the order in which the components are actuated.

Referring to FIGURE 1, an exemplary wound suturing apparatus 10 is shown having a housing 12, a tissue engaging portion 14, a shaft 16 extending from an opening 18 in the housing to the tissue engaging section 14, and a flexible guide tube 20 coupled at 22 to the tissue engaging section 14. The housing 12 has a body shaped like a pistol having a handle portion 24, and is illustrated in the exemplary embodiment as a two-piece construction of molded plastic. The apparatus 10 includes a pair of needles 26 and 28, which extend from housing 12 through the shaft 16 into the tissue engaging section 14. Each needle 26 and 28 has a non-tissue engaging end in the housing having a spherical member 30 and 32, such as a ball or bearing, respectively, attached thereto. Both needles 26 and 28 and spherical members 30 and 32 may be a made of metal, such as surgical stainless steel. The spherical members 30 and 32 may have a bore into which the non-tissue engaging ends of the needles 26 and 28, respectively, extend and joined thereto, such as by welding.

The apparatus 10 includes an actuator member 34 having two pins 36 extending into holes in the sides of housing 12 upon which the actuator member is pivotally mounted in the housing. Actuator member 34 has a portion that extends through an opening 38 in housing 12 to provide a trigger 40. A coil spring 42 is provided which hooks at one end in a notch 44 of actuator member 34 and is wound at the other end around a pin 46 located in holes in the sides of housing 12, such that the actuator member 34 is spring biased to retain trigger 40 normally in a forward position, as shown for example in FIG. 1. A notch 48 is provided in the actuator member 34 which is shaped to receive one of the non-engaging ends of needles 26 or 28, i.e., spherical members 30 or 32, to be driven forward by the actuator member 34 by a user pulling the trigger portion 40 of actuator member 34 towards handle portion 24. Two grooves 50 are provided by three fingers 52 into which portions of the needles 26 or 28 proximate to the spherical members 30 or 32, respectively, may lie.

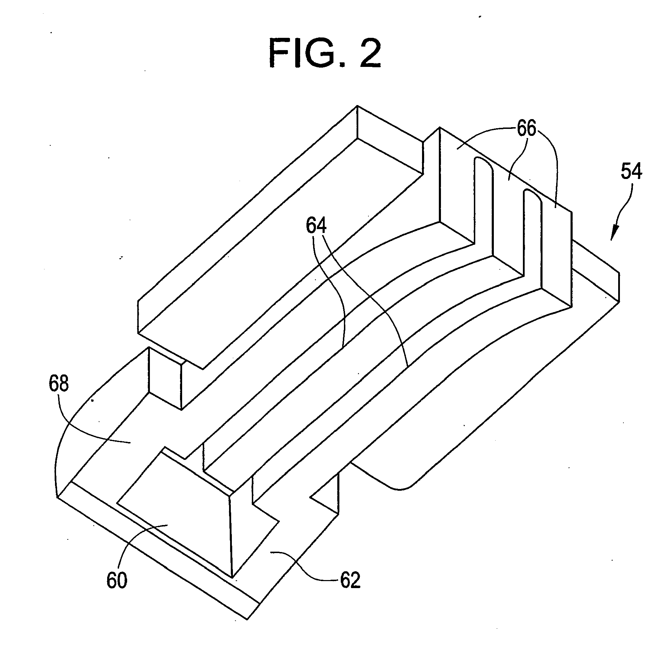

A retainer member 54 is fixed in housing 12 by two flanges 56, 58 above the actuator member 34 within mating surfaces 57. As best shown in FIGURE 2, the retainer member 54 has a chamber 60 having a lower opening 62 and two grooves 64 formed by fingers 66 which allow the spherical members 30 or 32 of needles 26 or 28, respectively, to be received in chamber 60 to restrict movement of the needle when held therein. The lower surface 68 of retainer member 54 is curved and faces correspondingly shaped fingers 52 of actuator member 34, such that the actuator member 34 is slidable along lower surface 68 responsive to a user pulling and releasing trigger 34.

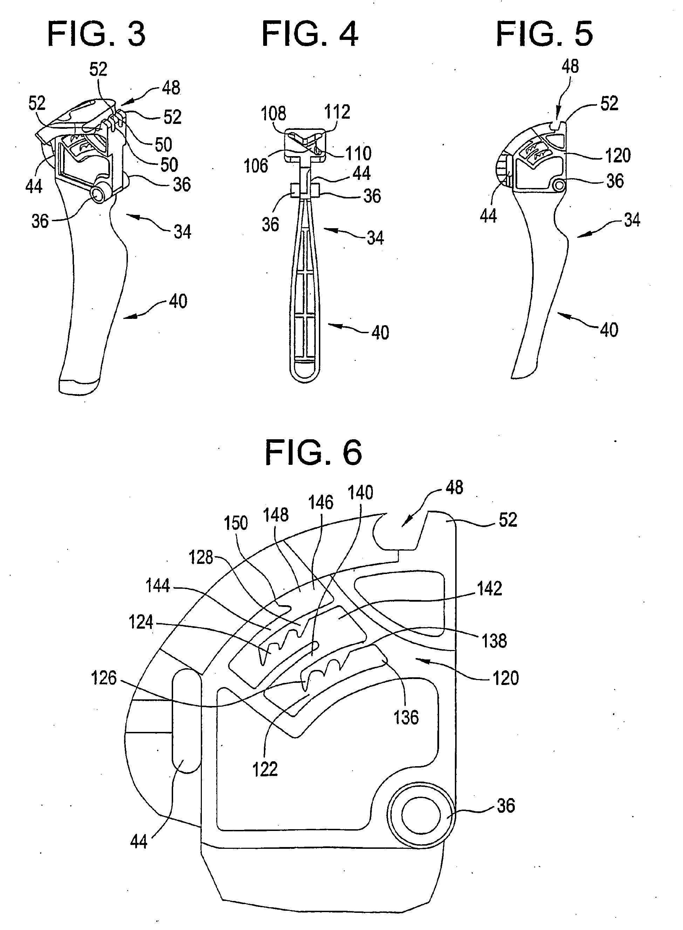

Referring now to FIGURES 3-6, an exemplary actuator mechanism 34 is shown in detail by the various perspective views. As described above, the actuator mechanism 34 includes a trigger portion 40, pins 36, a notch 44 for receipt of a coil spring 42, a notch 48 shaped to receive one of the non-engaging ends of needles 26 or 28, and grooves 50 provided by three fingers 52 into which portions of the needles 26 or 28 proximate to the spherical members 30 or 32, respectively, may lie.

Referring again to FIGURE 1, a needle selection mechanism is provided including a selector lever (or arm) 80, which is rotationally coupled with a cam member 82. The cam member 30 is supported by an adapter 84 in housing 12. The cam member 82 is mounted in housing 12 by two flanges 86, 88. The selector lever 80 is pivotally mounted by a pin 90 extending downwards from a distal end portion of the selector lever into a notch 92 in the housing 12. The selector lever 80 has a downwardly protruding member 94 which is received in a notch 96 of cam member 82 to rotate cam member 82 in a pocket between flanges 86 and 88 as the selector lever 90 is moved left or right. The cam member 82 has a tapered surface 98 to facilitate its rotation in pocket and two tapered apertures 100 and 102 through which needles 26 and 28 respectively extend. The selector lever 80 further includes a proximal pin 104 configured to engage a slot, shown generally at 106 on an upper portion of the actuator member 34.

In an initial configuration, the proximal pin 104 is positioned in a left lobe 108 of slot 106. During an initial actuation of the trigger portion 40, the proximal pin 104 of the selector mechanism 80 travels to trough 110 of the slot 106. During this actuation, the orientation of the cam member 82 is such that the needle 28 is in an engaged position within the grooves 50,48 of the actuator member 34 while the needle 26 is disengaged from the grooves 50, 48. The lobes 108, 112 and trough 110 of the slot 106 is configured such that release of the actuated trigger portion 40 causes the proximal pin 104 to travel into the right lobe 112 of the slot 106. This causes the proximal portion of the selector mechanism 80 to shift to the right, and at the same time, causes the cam member 82 to rotate in the same direction. The cam member 82 urges the needle 28 out of the grooves 50, 48 in the actuator mechanism 34 and simultaneously urges the needle 26 into the grooves 50, 48 in the actuator mechanism 34. Thus, a second actuation of the trigger portion 40 urges needle 26 in a distal direction but does not actuate the needle 28.

Referring now to FIGURES 5 and 6, the actuator mechanism 34 is provided with a slip-free mechanism, shown generally at 120. The slip-free mechanism 120 generally comprises dual ratchet tracks 122, 124, each with a plurality of ratchet teeth 126, 128. The ratchet tracks and teeth are configured to engage a traveling pin portion 130 of spring 132 (shown in FIGURE 1). Referring to FIGURE 6, the traveling pin portion 130 resides initially at a distal region 136 of ratchet track 122 and is biased against an upper wall 138 of the ratchet track 122. During initial actuation of the trigger portion 40 of the actuation member 34, the traveling pin portion 130 traverses the ratchet teeth 126. After a full actuation of the trigger portion 40, the traveling pin portion 130 moves into a first return channel 140. Release of the trigger portion 40 positions the traveling pin portion 130 at a distal region 142 of the second ratchet track 124. During a second actuation of the trigger portion 40, the traveling pin portion 130 traverses the ratchet teeth 128. After a full second actuation of the trigger portion, the traveling pin portion 130 moves into a second return channel 144. Release of the trigger portion 40 positions the traveling pin portion 130 at a distal region 146 of a lockout track 148. The traveling pin portion 130 is thereafter prevented from further movement by lockout stop 150.

Thus, the slip-free mechanism 120 and the traveling pin portion 132 of the spring 130 allow for only two actuations of the actuator mechanism 34. At the same time, the ratchet teeth 126 and 128 within the ratchet paths 122 and 124 prevent partial actuation of the actuator mechanism 34 and thus, partial deployment of the needles 26, 28.

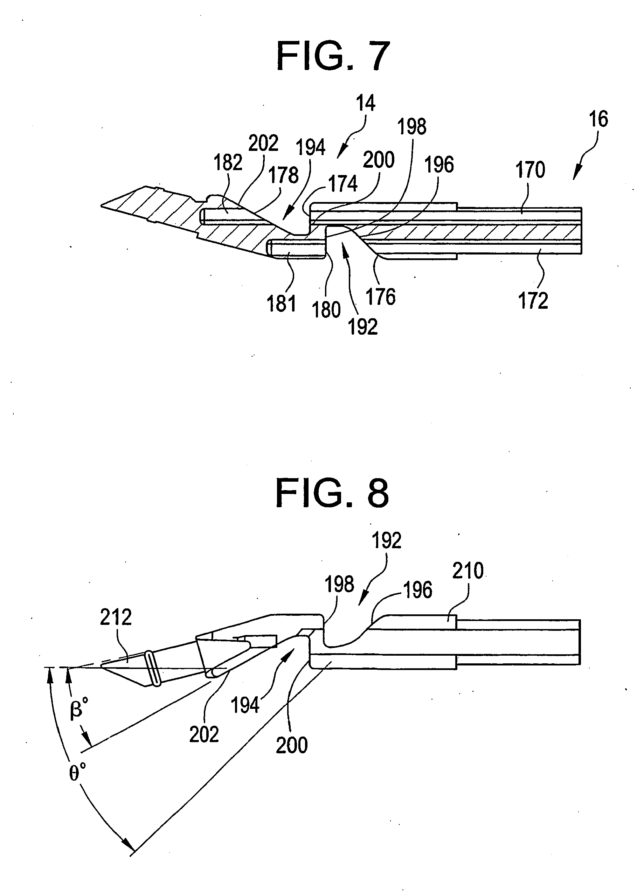

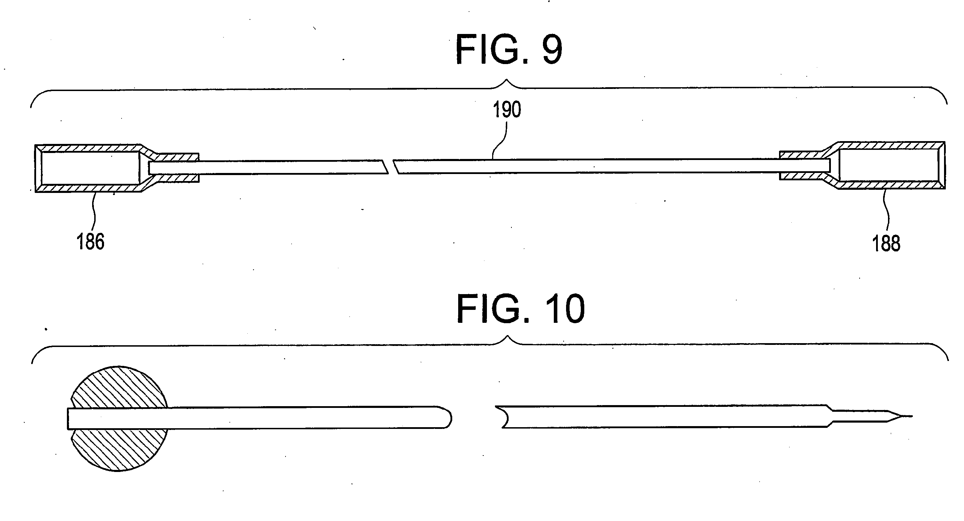

Referring to FIGURE 7, to orient the needles 26 and 28 within the tissue engagement section 14, the two needles 26 and 28 are configured to travel through generally parallel tracks 170, 172 in an x-z plane as they exit the shaft 16 and cross the tissue engaging section 14. To maximize the separation of the needles 26 and 28, the shaft 16 is oval in cross-section, having a major axis of the oval (though the cross-section may be circular or any convenient shape) for at least a substantial portion of the shaft as it extends to shaft end near the tissue engaging section 14. The tissue engaging section 14 of the tissue suturing apparatus 12 further includes a first opening 174, a second opening 176, and third 178 and fourth 180 openings providing access to distal channels 182 and 184, which are each capable of holding a needle capturing portion 186 and 188, respectively (see FIGURE 9), received through openings 178 and 180, respectively. Needle capturing portions 58a and 60a are referred to herein as ferrules, such as described, for example, in U.S. Patent Nos. 5,431,666 and 5,766,183, but may be any means by which a suture may be captured at the tip of a needle. The ferrules 186 and 188 each have an opening to an interior cavity shaped to enable the ferrule to frictionally engage the end of the needles 26 and 28, respectively, when received in the interior cavity. Each ferrule may be made of metal or plastic and may be oval in cross-section such that they can frictionally engage the tip of a needle. The ferrules 186 and 188 are each connected to one end of the two ends of a length of suture material or thread 190 extending through a suture tube or channel (not shown) positioned either in the elongated body 16 or in the flexible member 20.

In another embodiment the ferrules 186, 188 includes an interior cavity with an angled cross section providing multiple lines of interference. In another embodiment, the interior cavities of the ferrules 186, 188 have triangular cross sections, providing three lines of interference during engagement of the ferrule interiors with the distal tips of the needles. In another embodiment, the interior cavities of the ferrules 186, 188 have square cross-sections, providing four lines of interference during engagement of the ferrule interiors with the distal tips of the needles.

With reference to FIGURES 7 and 8, the tissue engaging section 14 has a first gap 192 and a second gap 194 in which the first gap 192 is along the lower side of section 14 and the second gap 194 is along the opposite upper side of section 14 and forward with respect to the first gap along the length of the section 14 in a direction distal from housing 12. The first gap 192 has two opposing surfaces 196 and 198 into which one side of a wound can be received, where opening 176 is located along surface 196 and opening 180 to ferrule holder 184 is located along surface 198 facing opening 176. Similarly, the second gap 194 has two opposing surfaces 200 and 202 into which the other side of the wound can be received, where opening 174 is located along surface 200 and the opening 178 to ferrule holder 182 is located along surface 202 and faces opening 174. Each gap 192 and 194 is shaped to have a depth to facilitate the placement of the edge of a wound therein. Surface 198, which is the distal face of the first gap 192, and surface 202, which is the proximal face of the second gap 194 both serve as stop surfaces for the tissue engaging section 14. Such stop surfaces 198, 200 assist in the placement of the tissue engaging section 14 relative to the wound as will be described further below. End portions 210 and 212 of the tissue engaging section are angled with respect to each other as shown in FIGURE 8 to facilitate placement of end 212 with guide section 20 through a sheath (or cannula) and the puncture wound to maximize blood vessel engagement. The two ferrules 186 and 188 and suture material 190 may be located in apparatus 10 during manufacture.

With reference to FIGURE 8, in one embodiment, stop surfaces 198 and 200 are squared at a middle portion thereof to provide good tactile feel to the surgeon of the stop points. In another embodiment, the stop surfaces may have an angle with regard to the longitudinal axis of the first end portion 210 of the tissue engaging section 14 of between about 85 and 95 degrees. In another embodiment, the stop surfaces have an angle of about 90 degrees.

Referring still to FIGURE 8, in one embodiment, wall 196 of the first gap 192 has an angle theta of between about 40 and 50 degrees with regard to the longitudinal axis of the first end portion 210 of the tissue engaging section 14. In another embodiment, wall 196 has an angle theta of about 45 degrees.

Referring still to FIGURE 8, in one embodiment, wall 202 of the first gap 194 has an angle beta of between about 25 and 35 degrees with regard to the longitudinal axis of the first end portion 210 of the tissue engaging section 14. In another embodiment, wall 202 has an angle beta of about 30 degrees.

Thus, by the above described exemplary ranges of the geometry of the tissue engaging section 14, an aggressive tissue contacting surface is described to facilitate bite of tissue, particularly for wound suturing devices having small sizes, where positive tissue capture and stop indication is particularly advantageous. In one embodiment, the tissue engaging section 14 has a size between about 2 and 2,67mm (6 and 8 French). In another embodiment, the tissue engaging section 14 has a size of about 2,33mm (7 French).

The tissue engagement section 14 may be made of metal, such as stainless steel, or other rigid biocompatible material. For example, the tissue engagement section may be made of two pieces of shaped metal having bores providing the desired openings, channels, and receptacles, joined together down the middle along section by welding or heat shrinking of heat shrinkable tubing connecting the two pieces. The components in the housing 12, such as the actuator member 34, selector lever 80, and needle retainer 220, may be made of molded plastic.

A guide section 20 is attached to end 212 (FIG. 8) of the tissue engaging section 14. As shown best in FIG. 1, the guide section 20 has a flexible tube 21 having an opening (not shown) through which a guide wire may be received. The tube 20 may be made of a biocompatible plastic, like heat shrink tubing, and the ramp may be made of plastic or metal, which is attached or joined within tube 20.

While the invention has been described with reference to an exemplary embodiment, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims.

## Claims

1. A suturing apparatus (10), comprising: a housing (12) and an elongated shaft (16) connected thereto; and two needles (26, 28) within said shaft (16) and two tissue engaging gaps (192, 194) on opposite sides of a tissue engaging section (14) positioned distally from said housing (12) on said shaft (16), wherein each needle (26, 28) is configured to travel distally across one of the tissue engaging gaps (192, 194), wherein each tissue receiving gap (192, 194) has two opposing surfaces (196, 198, 200, 202) into which one side of a wound can be received, wherein each gap (192, 194) is shaped to have a depth to facilitate the placement of the edge of a wound therein such that at least one surface of each gap (192, 194) comprises a stop surface (198, 202), **characterized in that** each stop surface (198, 202) is squared in relation to the longitudinal axis of the tissue engaging section (14) at a middle portion thereof to provide good tactile feel to the surgeon when tissue is engaged.

2. The suturing apparatus (10) of claim 1, wherein said stop surface (198, 202) has an angle relative to the longitudinal axis of a tissue engaging section (14) of the suturing apparatus (10), and wherein said angle is between about 85 and 95 degrees.

3. The suturing apparatus (10) of claim 1, wherein a second, opposing wall (196, 200) of said tissue receiving gap (192, 194) has an angle facilitating sliding of tissue across said second wall (196, 200).

4. The suturing apparatus (10) of claim 3, wherein said angle is between about 40 and 50 degrees relative to the longitudinal axis of the tissue engaging section (14).

5. The suturing apparatus (10) of claim 3, wherein said angle is between about 25 and 35 degrees relative to the longitudinal axis of the tissue engaging section (14).

6. The suturing apparatus (10) of claim 1, wherein said tissue engaging section (14) has a size of between about 2 and 2,67 mm (6 and 8 French).

7. The suturing apparatus (10) of claim 1, further comprising at two ferrules (186, 188) connected to one end of two ends of a length of suture material or thread, the ferrules (186, 188) positioned distally from each of the needles across said tissue engaging gaps.

8. The suturing apparatus (10) of claim 7, wherein said ferrules (186,188) have an interior cavity shaped according to one of an oval, triangular and square cross-section.

## Patentansprüche

1. Wunden-Nähvorrichtung (10) aufweisend: ein Gehäuse (12) und ein damit verbundenen länglichen Schaft (16); und zwei Nadeln (26, 28) innerhalb des Schafts (16) und zwei Gewebeeingriffslücken (192, 194) auf gegenüberliegenden Seiten eines Gewebeeingriffsabschnitts (14), welcher distal von dem Gehäuse (12) auf dem Schaft (16) positioniert ist, wobei jede Nadel (26, 28) so gestaltet ist, dass sie in distaler Richtung durch eine der Gewebeeingriffslücken (192, 194) läuft, wobei jede Gewebeaufnahmelücke (192, 194) zwei gegenüberliegende Flächen (196, 198, 200, 202) aufweist, in die eine Seite einer Wunde aufgenommen werden kann, wobei jede Lücke (192, 194) so gestaltet ist, dass sie eine Vertiefung aufweist, um die Aufnahme des Rands einer Wunde zu erleichtert, so dass zumindest eine Fläche jeder Lücke (192, 194) eine Anschlagfläche (198, 202) aufweist, **dadurch gekennzeichnet, dass** jede Anschlagfläche (198, 202) in Bezug auf die Längsachse des Gewebeeingriffsabschnitts (14) im rechten Winkel steht, um ein gutes Tastgefühl für den Chirurgen zu verschaffen, wenn das Gewebe im Eingriff steht.

2. Wunden-Nähvorrichtung (10) gemäß Anspruch 1, **dadurch gekennzeichnet, dass** die Anschlagfläche (198. 202) in einen Winkel relativ zur Längsachse eines Gewebeeingriffsabschnitts (14) der Nähvorrichtung (10) steht, wobei der Winkel zwischen etwa 85° und 95° ist.

3. Wunden-Nähvorrichtung (10) gemäß Anspruch 1, **dadurch gekennzeichnet, dass** eine zweite, gegenüberliegende Wand (196, 200) der Gewebeaufnahmelücke (192, 194) einen Winkel aufweist, der das Gleiten des Gewebes über die zweite Wand (196, 200) erleichtert.

4. Wunden-Nähvorrichtung (10) gemäß Anspruch 3, **dadurch gekennzeichnet, dass** der Winkel etwa zwischen 40° und 50° relativ zur Längsachse des Gewebeeingriffsabschnitts (14) ist.

5. Wunden-Nähvorrichtung (10) gemäß Anspruch 3, **dadurch gekennzeichnet, dass** der Winkel etwa zwischen 25° und 35° relativ zur Längsachse des Gewebeeingriffsabschnitts (14) ist.

6. Wunden-Nähvorrichtung (10) gemäß Anspruch 1, **dadurch gekennzeichnet, dass** der Gewebeeingriffsabschnitt (14) eine Größe zwischen etwa 2 mm und 2,67 mm aufweist.

7. Wunden-Nähvorrichtung (10) gemäß Anspruch 1, **dadurch gekennzeichnet, dass** sie ferner zwei Hülsen (186, 188) aufweist, die mit einem Ende von zwei Enden eines Nahtmaterials oder Fadens verbunden sind und die Hülsen (186, 188) distal von den durch die Gewebeeingriffslücken verlaufenden einzelnen Nadeln positioniert sind.

8. Wunden-Nähvorrichtung (10) gemäß Anspruch 7, **dadurch gekennzeichnet, dass** die Hülsen (186, 188) eine innere Aussparung aufweisen, die nach einem ovalen, dreieckigen und viereckigen Querschnitt gestaltet ist.

## Revendications

1. Appareil à suturer (10) comprenant : un boîtier (12) et un arbre allongé (16) qui lui est connecté ; et deux aiguilles (26, 28) dans chaque arbre (16) et deux intervalles d'engagement de tissu (192, 194) sur les côtés opposés d'une section d'engagement de tissu (14) positionnée dans le sens distal en partant dudit boîtier (12) sur ledit arbre (16), chaque aiguille (26, 28) étant configurée pour se déplacer dans le sens distal en travers d'un des intervalles d'engagement de tissu (192, 194), chaque intervalle récepteur de tissu (192, 194) comportant deux surfaces opposées (196, 198, 200, 202) dans lesquelles peut être reçu un côté d'une plaie, chaque intervalle (192, 194) étant conformé avec une profondeur facilitant le placement du bord d'une plaie dedans, de sorte qu'au moins une surface de chaque intervalle (192, 194) comprend une surface d'arrêt (198, 202), **caractérisé en ce que** chaque surface d'arrêt (198, 202) est carrée en relation avec l'axe longitudinal de la section d'engagement de tissu (14) au niveau d'une de ses parties médianes pour permettre une bonne sensation tactile du chirurgien lorsque du tissu est engagé.

2. Appareil à suturer (10) selon la revendication 1, dans lequel ladite surface d'arrêt (198, 202) forme un angle par rapport à l'axe longitudinal d'une section d'engagement de tissu (14) de l'appareil à suturer (10) et ledit angle est compris entre 85 et 95 degrés.

3. Appareil à suturer (10) selon la revendication 1, dans lequel une seconde paroi opposée (196, 200) dudit intervalle récepteur de tissu (192, 194) forme un angle facilitant le coulissement de tissu à travers ladite seconde paroi (196, 200).

4. Appareil à suturer (10) selon la revendication 3, dans lequel ledit angle est compris entre 40 et 50 degrés par rapport à l'axe longitudinal de la section d'engagement de tissu (14).

5. Appareil à suturer (10) selon la revendication 3, dans lequel ledit angle est compris entre 25 et 35 degrés par rapport à l'axe longitudinal de la section d'engagement de tissu (14).

6. Appareil à suturer (10) selon la revendication 1, dans lequel ladite section d'engagement de tissu (14) a une dimension comprise entre environ 2 et 2,67 mm (6 et 8 pouces).

7. Appareil à suturer (10) selon la revendication 1, comprenant en outre deux ferrules (186, 188) connectées à une des deux extrémités d'une longueur de matériau ou fil de suture, les ferrules (186, 188) étant positionnées dans le sens distal en partant de chacune des aiguilles en travers desdits intervalles d'engagement de tissu.

8. Appareil à suturer (10) selon la revendication 7, dans lequel lesdites ferrules (186, 188) ont une cavité intérieure conformée suivant une section transversale parmi celles de forme ovale, triangulaire et carrée.
